(19) Europäisches Patentamt
European Patent Office
Office européen des brevets

(11) EP 1 790 349 A1

(12) EUROPEAN PATENT APPLICATION
published in accordance with Art. 158(3) EPC

(43) Date of publication:
**30.05.2007 Bulletin 2007/22**

(21) Application number: **05779458.8**

(22) Date of filing: **02.09.2005**

(51) Int Cl.:
***A61K 33/22*** *(2006.01)*

(86) International application number:
**PCT/MX2005/000079**

(87) International publication number:
**WO 2006/025724 (09.03.2006 Gazette 2006/10)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **02.09.2004 MX PA04008485**

(71) Applicant: **Galvan Perez, Juan Pablo**
**CEP-01460 Mexico D.F. (MX)**

(72) Inventor: **Galvan Perez, Juan Pablo**
**CEP-01460 Mexico D.F. (MX)**

(74) Representative: **Viering, Jentschura & Partner**
**Grillparzerstrasse 14**
**81675 München (DE)**

# (54) IMPROVEMENTS TO SYNTHESIS, PHARMACEUTICAL COMPOSITIONS AND USES OF SODIUM PENTABORATE PENTAHYDRATE (NaB5O8.5H2O)

(57) The invention refers to an improved method for the synthesis of pentahydrated sodium pentaborate $NaB_5O_8.SH_2O$; the method consists in reacting disodium tetraborate decahydrate (DTBD, Borax) and orthoboric acid (OBA, boric acid). The process is performed in no longer than five days and it does not require fungal strains . It also refers to the product obtained by this method, which is intended to potentiate the immune system in diseases caused by bacteria and viruses; to its pharmaceutical compositions and to its use in the treatment of these diseases.

EP 1 790 349 A1

## Description

## FIELD OF THE INVENTION:

The invention discloses an improved process to elaborate a cyclic compound of boron and sodium, the compounds containing the same, and new uses of the compound referring to immuno-modulatory (or immunomodulating) effect in mammals.

## BACKGROUND:

It is generally accepted that the Human Immunodeficiency Virus type I (HIV-1) is the agent causing AIDS. Since the first AIDS case was reported in 1981 at the Centers for Disease Control (CDC), more than 10,000 cases have been reported in the United States. More than 50% of these patients have already died. Based on the estimates, from one to two million Americans are nowadays infected with HIV, while being asymptomatic, 20-30% of them will develop the disease in five years; by 2008, there will be nearly 1,300,000 symptomatic patients, and about 10,000 of them will be children. The viral nature of the disease has boosted research on HIV-1 inhibitors. By virtue of the inhibitory effect on the viral replication cycle it can be expected that these inhibitors will prevent the progression of the disease and possibly promote the recovery of the patient. Additionally to this approach and as HIV causes weakening of the immune system, several immunomodulators have been investigated. It can be thought that an agent capable of boosting the antiviral immune response will help eliminating the virus.

The validity of the antiviral therapy in the induced HIV-1 disease was apparent when demonstrating that suramin, a potent retroviral inhibitor, in association with a reverse transcriptase, suppressed HIV-1 replication *in vivo* and *in vitro.* Nevertheless, a clinical or immunological improvement was not observed after a six-week treatment period, because the treatment had to be ended due to the toxicity of the drug. A longer treatment (37 weeks) did not produce a clinical or immunological improvement.

The following investigated compound was AZT (3'-azido-2',3'-dideoxytimidine), which inhibited the HIV-1 at significantly lower concentrations with significantly higher inhibitory levels than those observed with Suramin. AZT treatment of both patients with AIDS and AIDS Related Complex (ARC) during a six-week period, produced clinical and immunological improvement as well as a reduction of the frequency of opportunist diseases and a reduction of the death rate during the 24-week therapy period. AZT, also known as Retrovir or Zidovudine, is the only drug approved by the FDA for the treatment of this disease. In spite of the fact that AZT has been beneficial for some AIDS or ARC patients treated for up to 24 weeks, the beneficial effects of the drug decline for a six-month period.

New drugs that seem to be promissory in the actual clinical assays include DDC (2', 3'-dideoxycitidine), DDI (2',3'dideoxyinosine) and DDA (2',3'-dideoxyadenosine). Among these three, DDI has shown no or low toxicity and has caused a significant clinical or immunological improvement.

Once the destruction of the immune system has occurred, the combination of an antiviral therapy and an immune response modulator such as interferon (HuIFN-alfa,-beta or -gamma) or a granulocyte and macrophage colony stimulator factor (GM-CSF) can be guaranteed. Usually, the immunomodulators are administered together with an antiviral agent. For example, it has been demonstrated that GM-CSF enhances the monocite production in HIV-1, but when used in combination with AZT, it enhances the entire AZT antiviral activity.

Up to date, the use of pentahydrated sodium pentaborate {$NaB_5O_2 .5H_2O$} has not been disclosed for the treatment of other viral or bacterial diseases.

## OBJECT OF THE INVENTION:

1.- To provide an immunomodulator by means of a faster manufacturing method than the one described in the state-of-the-art.

2.- To provide a fast method to elaborate a potentiating substance for the immune system.

3.- To propose the use of immunomodulators to potentiate and boost the immune system in certain diseases involving viruses, including AIDS.

4.- To use the immunomodulator of the invention, for the treatment of several diseases involving viruses.

5.- To propose the use of an immunomodulator to potentiate and boost the immune system in several bacterial diseases.

6.- To use the immunomodulator of the invention, to treat several diseases involving bacteria.

## BRIEF DESCRIPTION OF THE DRAWING

**Figure 1:** Nuclear magnetic resonance spectrum of $^{11}B$ in deuterated water.

## DETAILED DESCRIPTION OF THE INVENTION

This invention is an improvement of the invention published in PCT application under international number PCT/US90/06331, published under number WO 91/06304 Dressman et al.; the application referrers to the synthesis, compounds and medical application of cyclic pentaborate. It referred to several chemical compounds derived from pentaborate and their application was exclusively targeted to the treatment of AIDS.
The aforementioned application claimed a very large process consisting in eight stages and required about 40 working days to obtain the pentaborate derivatives, which were later used in the treatment of human immunodeficiency syndrome.
The current invention consists in a method taking no longer than five days to obtain a single product addressed to potentiate the immune system during treatment of diseases caused by bacteria and viruses, to the method for obtaining the same and to new uses, which are considered as an improvement of the above-mentioned invention of said application.
The synthesis method described below is used to obtain pentahydrated sodium pentaborate, from now on PSP. This method enables to obtain the mentioned compound in no more than five days, while by means of the synthesis method of patent application PCT/US90/0633, at least 40 days are required.
The raw material used for this synthesis method is disodium tetraborate decahydrate (Borax or DTBD), orthoboric acid (OBA, boric acid), both provided by U.S. Borax, and purified water.

DISODIUM TETRABORATE DECAHYDRATE ASSAY

REFERENCE: U.S. Pharmacopoeia XXII/NF XVII

OBJECTIVE: To determine the purity of disodium tetraborate decahydrate (DTBD) as raw material in the method for obtaining the PSP described herein.
PRINCIPLE: DTBD in aqueous solution is titrated up to the end point of methyl red with standardized hydrochloric acid.

EQUIPMENT

50-mL burette
Magnetic agitator with stirring bar
250-mL beaker
Analytical scale with milligram accuracy

CHEMICALS

Hydrochloric acid (HCl) 0.5 N standardized against sodium hydroxide standard.
0.1% methyl red indicator solution in methanol.

PROCEDURE

1. Weight about 3.0 g of a DTBD sample, the closest possible to the milligram. Record weight. Add this sample to 50 mL purified water into a 250-mL beaker with a stirring bar, stir over a magnetic stirrer for 10 to 15 minutes to obtain a saturated solution. Add 5 drops of the methyl red indicator solution. Titrate with standardized HCl 0.5 N. Add the initial 30 mL of titrating solution by dripping it as fast as possible while the pH of the solution is maintained above the end point (pH 4.8). Allow any remaining sodium DTBD crystal to dissolve. Keep dripping the titrant slowly until the end point. Record the total HCl 0.5 N volume required.
2. Repeat step No. 1 with two additional 3.0 g samples of DTBD. Record every result.

VALIDATION OF THE ASSAY FOR DISODIUM TETRABORATE DECAHYDRATE $Na_2B_4O_7 . 10H_2O$ (DTBD) AS RAW MATERIAL EQUIPMENT

25-mL Kimax$^R$ Schellbach Automatic Burette, Kimble 17170F.
Mettler analytical scale model H54
Cole-Palmer stirrer/hot plate, model 4658.
CHEMICALS: The following chemicals were used; trademarks and specifications are indicated.
Disodium tetraborate decahydrate, EM Suprapur$^R$ No. 6309-1, batch 9118.
Sodium hydroxide 0.5 N prepared from the 1 N solution by VWR by Biotech Resourcess Inc. (BTR) batch 90072601
Hydrochloric acid 0.5 N, prepared from the 2 N solution of RICAA, batch No. 90060601 by BTR
Potassium biphtalate (KHP), Baker analytical grade, standard acidimetry No. 2958-01, batch D18084.
Methyl red, sodium salt, Baker analytical grade No. R086-02, batch D14708
Water was obtained from the Continental Water Systems Modulab unit, with a resistivity of more than 18 Megohm-cm.
PROCEDURE: Sodium hydroxide was standardized in tripticate using 3.000 g potassium biphtalate aliquots, which were oven dried at 115°C for one hour and stored in a desiccator until used.
Hydrochloric acid was standardized in tripticate using 10-mL aliquots of standardized NaOH 0.5 N.
The titration was performed in tripticate using 3.000 g of the sample per determination (Suprapur®) unless otherwise specified.

CALCULATIONS

$$\text{Purity (P)} = \frac{\text{Titrated DTBD weight.}}{\text{Weight of the sample}} \times 100$$

Titrated DTBD weight= DTBD eqs. X DTBD eq. weight
Eqs.= equivalents
Eq. = equivalent
DTBD equivalents = (mL HCl) (N HCl)/1000

$$P = \frac{\text{(mL HCl) (N HCl) (DTBD Eq. weight)}}{\text{Sample weight x 1000}} \times 100$$

Each mL of HCl 0.5 N is equivalent to 95.34 mg of $Na_2B_4O_7 .10H_2O$, hence the equation can be expressed as follows:

$$P = \frac{\text{(mL HCl) (N HCl)}}{\text{Weight of the sample}} \times 95.34$$

Weight of the sample
For anhydrous DTBD, e.g. Suprapur$^R$

$$P = \frac{(mL\ HCl)(N\ HCl)}{\text{Weight of the sample}} \times 10.06$$

DTBD purity must be between 99.0 and 105.0%

RESULTS: The following data are the actual results obtained in laboratory and are mentioned as examples.

A. Acid-base standardization

1. NaOH 0.5 N, batch 90072601 titrated 5X with 3 g KHP 0.5 N (5X)
2. HCl 0.5 N, batch 90060601 titrated with NaOH 0.5 N, batch No. 90072601, NaOH normality 0.501, 0.500, 0.502; mean 0.501 N

B. Accuracy verified with anhydrous DTBD EM Suprapur$^R$

EXAMPLE 6: 4 DTBD samples were weighted the closest to 3.000 g; the purity of each sample was calculated by the methods above described and with the equations above mentioned.

| Weight of the sample | mL HCl 0.5 N | % DTBD (g) purity |
|---|---|---|
| 3.000 | 59.23 | 99.51 |
| 3.000 | 59.32 | 99.66 |
| 1.584 | 31.27 | 99.50 |
| 1.580 | 31.28 | 99.78 |
| | Average | 99.61 |

Note: The last two determinations were lower than 3.00 grams, due to insufficient of sample.

In like manner, the purity of each of the three samples was calculated from different DTBD batches, before its use as raw material in the method to obtain PSP in the present invention, results are summarized in Table 1.

TABLE 1. DATA FROM EXAMPLES 7, 8, AND 9

| Example | Batch | Purity (%) | | | Average (%) |
|---|---|---|---|---|---|
| 7 | 11299-8 | 101.6 | 101.5 | 101.5 | 101.5 |
| 8 | 11029-8 | 103.0 | 103.0 | 104.9 | 103.6 |
| 9 | 10249-8 | 104.5 | 103.6 | 105.0 | 104.4 |

ORTHOBORIC ACID ASSAY

REFERENCE: U.S. PHARMACOPOEIA XXII/National Formulary XVII, p. 1906

OBJECTIVE: To determine the purity of the orthoboric acid that will be used in the production of PSP.

PRINCIPLE: The sample is titrated with standard sodium hydroxide in the presence of glycerol. Glycerol forms a complex with orthoboric acid to make it more soluble, and to increase the end point, and improve accuracy.

EQUIPMENT

250-mL beaker

Magnetic stirrer and stir bar.

50-mL burette

Pasteur pipettes

Analytical scale (milligram accuracy)

CHEMICALS

Standard 1 N sodium hydroxide solution (NaOH 1 N) Glycerol

0.1 N Sodium hydroxide solution 0.1 N (NaOH 0.1 N)

1% Phenolphthalein alcohol solution as indicator

PROCEDURE

1. Carefully mix 50 mL of glycerol and 50 mL of deionized water into a 250-mL flask using a stirring bar over a magnetic stirrer. Add 5 drops of phenolphthalein solution as indicator. Adjust the pH to the "phenolphthalein end point" adding a few drops of NaOH 0.1 N; record the number of drops required for the titration. The most important factor when neutralizing the glycerol is consistency, more than accuracy. Only a few drops of NaOH 0.1 N are required (1-3). This value is constant for a determined glycerol value.
2. Weight about 2.0 g of orthoboric acid from the sample, accuracy closest to a milligram. Record the weight. Add this sample into the 50% glycerol water sample and start titration with standard NaOH 1 N. Add 30 mL of NaOH to promote dissolution of orthoboric acid. Stir until all orthoboric acid has completely dissolved; no more than 15 minutes. Continue titrating until reaching phenolphthalein end point. Record the volume of standard NaOH 1 N required, closest to 0.01 mL. 32.35 mL of standard NaOH 1 N are required for the titration of 2.000 g orthoboric acid 100% pure.
3. Add additional 50 mL of glycerol to challenge the complex formation of orthoboric acid. Add the number of drops of NaOH 0.1 N as recorded in step 3, required to neutralize glycerol. Continue with the titration with standard NaOH 1 N. Record the final volume of the required titrating solution, closest to 0.01 mL.
4. Repeat steps 3 to 5, with two additional 2.0 g samples of orthoboric acid. Record all obtained results.

Orthoboric acid must be between 99.5 and 100.5% pure.

VALIDATION OF ORTHOBORIC ACID ASSAY AS RAW MATERIAL. EQUIPMENT

50-mL burette class B Kimax

Mettler analytical scale model H54

Cole-Palmer stirrer/hot plate model 4658

CHEMICALS: The following chemicals were used; trademarks and specifications are shown.

Orthoboric acid EM Suprapur$^{R}$ No. 765-1, batch No. 9249.

Sodium hydroxide 1.0 N from RICCA, No. 7450, batch No. B071, prepared by BTR batch 01382

Potassium biphtalate Baker, analytical grade, standard acidimetry No. 2958-01, batch No. D18084.

Phenolphthalein, EM Science No. PX 0525-1, batch No. 9142, 1% solution in ethanol.

Glycerol Baker analytical grade No. M778-09, batch C33610.

Water was obtained from the Continental Water Systems Modulab unit with a resistivity of more than 18 Megohm-cm.

PROCEDURE: Sodium hydroxide was standardized in tripticate to 6.000 g potassium biphtalate aliquots, and oven dried at 115°C for one hour and stored in a desiccator until used. The titration was performed in tripticate, using 2.000 g of the sample by determination.

CALCULATION:

$$\text{Purity (P)} = \frac{\text{Weight of the titrated orthoboric acid.}}{\text{Weight of the sample}} \text{ X } 100$$

Weight of the titrated orthoboric acid = Equivalents of orthoboric acid x equivalent weight of the orthoboric acid. Equivalents of orthoboric acid = (mL NaOH)(N NaOH)/1000
Eqs.= equivalents
Eq. = equivalent
Replaced into the equation to calculate purity.

$$P = \frac{(\text{mL NaOH})(\text{N NaOH})\ \text{Eq. weight orthoboric acid}}{\text{Sample weight} \times 1000} \times 100$$

Each mL of NaOH 1 N is equivalent to 61.83 mg of orthoboric acid ($H_3BO_3$), hence the equation can be written as follows:

$$\text{Purity} = \frac{(\text{mL NaOH})(\text{N NaOH})}{\text{Weight of the sample}} \times 6.183$$

Orthoboric acid must be between 99.5 and 99.8% pure.
RESULTS: The following data are the actual results obtained in laboratory and are introduced as examples.

A. Sodium hydroxide standardization.

NaOH 1 N batch 01382 was used to titrate 6 aliquots of 6 grams of potassium biphtalate (3X): 1.007 N. B. The accuracy of the orthoboric acid batch 9249, was verified with EM Suprapur[R]
EXAMPLE 1: 3 orthoboric acid samples were weighted closest to 2.000 g; the purity of each sample was calculated by the methods above described and with the equations above mentioned.

| Weight of the sample of Orthoboric acid. | % purity |
|---|---|
| 2.003 | 100.52 |
| 2.003 | 100.52 |
| 2.004 | 100.49 |
| Average | 100.51 |

The same way, the purity of each of the three samples was calculated from different orthoboric acid batches, before its use as raw material in the method to obtain PSP in the present invention. Results are shown in Table 2.

TABLE 2 RESULTS OF THE ANALYSIS OF PURITY OF SOME ORTHOBORIC ACID SAMPLES.

| Example | Batch | Purity (%) | | | Average (%) |
|---|---|---|---|---|---|
| 2 | 10239-7 | 100.18 | 100.21 | 100.21 | 100.20 |
| 3 | 11099-7 | 100.27 | 100.34 | 100.40 | 100.34 |
| 4 | 12079-7 | 100.40 | 100.43 | 100.43 | 100.42 |
| 5 | 10099-7 | 100.40 | 100.28 | 100.33 | 100.34 |

METHOD OF SYNTHESIS OF PENTAHYDRATED SODIUM PENTABORATE (PSP) {$NaB_5O_8.H_2O$} TO OBTAIN ONE KILOGRAM OF THE PRODUCT IN LABORATORY. EQUIPMENT

3000-mL beaker with handle

108x38-mm stir bar, floating Teflon, Nalgene trademark
Scale, Ohaus GT4000
Weighing dish (2)
2000-mL graduated glass cylinder
Hot plate stirrer, Corning PC 520 (2)
Plastic container, 1/3-cup capacity (2) -20 to 150°C thermometer
Corning pH-meter 220 with combination electrode 24-cm Büchner funnel
Vacuum bomb with gauge
4-L Kitasato
Whatman filter paper No. 41, 24-cm
Whatman filter paper No. 1, 24-cm
5-L plastic container, Rubbermaid
Plastic stir bar
Plastic spatula
Water bath plastic container
1000-mL wide mouth bottle Nalgene PMP with screw cap (2)
125-mL narrow mouth bottle Nalgene with screw cap
Fiberglass tray, MFG

CHEMICALS

Disodium tetraborate decahydrate (DTBD, borax), U.S. Borax, NF
Orthoboric acid (OBA, boric acid), U.S. Borax, NF
Purified water

PROCEDURE

I. PREPARATION OF THE CONTAINER

A. 3000-mL Pyrex beaker is labeled.
B. 108x38 mm stir bar is placed inside.

II. WEIGHING PROCEDURE

A. The calculated quantity of disodium tetraborate decahydrate (DTBD) is weighted (equivalent to approximately 700.0 grams of 100% pure) in a weighing tared container.
B. Orthoboric acid is weighted, equivalent to approximately 680.0 grams orthoboric acid 100% pure, in a weighing tared container.

III. PREPARATION OF THE SOLUTION

A. Add approximately 1600 mL of purified water into the reaction beaker.
B. Heat water in a Cole-Palmer model 4658 hot plate stirrer, set to heating level 5; with the thermometer, check the temperature from time to time.
C. When temperature reaches 57°C to 59°C, reduce the heating level to 4. While slowly stirring at 200 RPM, add alternately the weighed DTBD and OBA, namely, first a DTBD portion and then an OBA portion, about 50 g each, once again a DTBD portion and so on until all chemicals are completely added, allow the added portion to completely dissolve before adding the next portion, without stopping the movement of the stirring bar. All chemicals are added in approximately 12 minutes.
D. The chemicals are mixed until complete dissolution. Do not allow the temperature to exceed 61°C. This is best controlled if the heating level is reduced to level 3 when temperature reaches about 55°C. Measure and record the pH of the hot solution, which must be 6.42. Take away from the hot plate and measure the pH once again, it must

be 6.37, let stand to cool down in a container; pH is around 6.20.

IV. COMPLETION OF THE PREPARATION

A. During cooling-down and when the pH is around 6.20, the hot solution is filtered thru a Whatman filter paper No. 41 in a 24-cm Büchner funnel, under 5 to 10 cm Hg vacuum. Remove the stirring bar and wash it with purified water.
B. Transfer the filtrate into a 5L-plastic container together with the stirring bar. Allow the solution to cool down to 33-34°C stirring at 150 RPM to promote crystal formation. This is done by any known method, but the cooling-down method is preferred. Transfer the container into a water bath at approximately 21°C and keep on stirring at 150 RPM. When temperature is between 25 and 27°C, add ice into the water bath, so that temperature decreases to 10-12°C, keep stirring slowly. At this point the solution is refrigerated.
C. Refrigerate the solution for 18-20 hours at about 6°C.
D. Collect the crystals in a 24-cm Whatman filter paper No. 1 under a negative pressure of 20-30 cm Hg (vacuum), allow the collected mass to dry under vacuum for 60-70 minutes.
E. Carefully of not harming the paper fibers so that they do not get mixed with the product, spread the crystals over a fiberglass tray and place in an oven at a temperature of around 35°C for 48 to 72 hours. Stir the mass periodically to allow drying.
F. Measure and record the volume of the collected filtrate. Take 100 mL in a plastic bottle for the qualitative analysis.
G. Collect and weigh the dry crystals. Theoretically, 1084 grams are obtained. Store the product in labeled polypropylene bottles with screw cap.

V. EXAMPLE OF THE LABORATORY PROCESS TO OBTAIN PSP.

| Time (minutes) | Solution temperature around (°C) | Action | Notes |
|---|---|---|---|
| 0 | 57 | Add about 50 g alternating each reactant. Reduce heating to 4. | Stir at around 200 RPM |
| 2 | 48 | | Addition of the first half of each reactant is complete. |
| 3 | 46 | Add about 50 g, alternating each reactant. | |
| 4 | 45 | Add about 50 g, alternating each reactant. | |
| 5 | 43 | Add about 50 g, alternating each reactant. | |
| 6 | 41 | Add about 50 g, alternating each reactant. | |
| 7 | 40 | Add about 50 g, alternating each reactant. | |
| 8 | 39 | Add about 50 g, alternating each reactant. | |
| 10 | 38 | Add about 50 g, alternating each reactant | All reactants have been added to water |
| 25 | 51 | Set heat to 3 | |
| 30 | 57 | | The reactants are completely dissolved |
| 35 | 61 | Remove from heat and record the pH | |

(continued)

| Time (minutes) | Solution temperature around (°C) | Action | Notes |
|---|---|---|---|
| 40 | 48 | When temperature is about 33-34 °C, transfer the filtrate into a 5L-plastic container and slowly begin stirring at setting 3. | |
| 45 | 45 | Slowly stir at around 150 RPM | No signs of crystal formation. |
| 55 | 42 | | Crystals start forming up. |
| 105 | 38 | Increase stirring speed to 4 (200 RPM) | |
| 150 | 34 | Transfer the container into a water bath, at around 21°C. | |
| 190 | 26 | Manual stirring Add ice into water bath | |
| 200 | 22 | Manual stirring | |
| 240 | 11 | Transfer to refrigerator | |

At industrial scale, the synthesis process will be as follows, per 100 kilograms of desired final product:

I. WEIGHTING PROCEDURE.

a. For 108.4 kilograms (theoretical) of product, the calculated quantities are weighed (approximately equivalent to 70.0 kilograms 100% pure disodium tetraborate decahydrate (DTBD)) in a suitable container and
b. In a different container weigh about 68.0 kilograms of orthoboric acid.

II. PREPARATION OF THE SOLUTION

a. Add about 160.0 L of purified water into a reaction container.
b. Heat water while stirring at 200 RPM, periodically checking the temperature with the thermometer.
c. When the temperature reaches 57-61°C, decrease heating so that the temperature keeps below 61°C. While stirring at 200 RPM, slowly add the weighed DTBD and OBA until they are completely dissolved, under continuous stirring.
d. Do not allow temperature to exceed 61°C. Measure and record the pH of the hot solution. pH must be 6.42.

III. COMPLETION OF THE PREPARATION

a. Filter the hot solution under vacuum, to eliminate solids.
b. Transfer the filtrate into a container and cool the solution down to about 10-12°C while stirring at 150 RPM.
c. Refrigerate the solution for 18-20 hours at about 6°C.
d. Collect the crystals using vacuum of 20-30 cm Hg; allow the collected mass to dry unperturbed in an oven at 35°C for 48-72 hours. Stir the mass periodically to allow drying.
e. Measure and record the volume of collected filtrate. Take 100 mL for the qualitative analysis.
f. Collect and weigh the dry crystals. Theoretically, 108.4 kilograms are obtained. Store the product in labeled polypropylene containers with screw cap.

This process takes less than five days to obtain the final product in dry crystals already packed, different from the PCT application with international number PCT/US90/06331, published as WO 91/06304 Dressman et al., that takes nearly 40 days. However, the industry has available equipments such as the Tender No 93-0113-A, Spray Dryer, Mobil Minor 2000, whereby the product can be obtained in the form of dry crystals within one day. PSP shows the following characteristics:

Condensed formula: $NaB_5O_8 .5H_2O$
The theoretical $NaB_5O_8 .5H_2O$ composition is:

$Na_2O$ (10.5%); $B_2O_3$ (59.0%); $H_2O$ (30.5%); calculated from the molecular weights published for the compounds and their components, expressed as the combination of sodium oxide, boron trioxide and water.

Figure 1 is the result of the analysis by nuclear magnetic resonance of $^{11}B$ in deuterated water, performed at CINVESTAV (Center for Research and Advanced Studies), Mexico, showing that PSP is a mixture mainly formed by sodium borate and boric acid.

Structure:

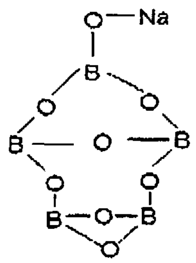

PENTAHYDRATE

Molecular weight: 295.17
Melting point: It does not melt. It dehydrates above 100°C, releases degradation products by sublimation and afterwards at higher temperatures, it breaks down.

IDENTIFICATION PARAMETERS

APPEARANCE: Fine white crystalline powder.
ASSAY; Contains more than 95% and no less than 105% {$NaB_5O_8 .5H_2O$}. This interval depends on some small variations of the hydration degree.
The humidity content as per a thermo gravimetric analysis is between 28.5 and 32.5%.
pH: 7.6 a 8.2 (4% solution)
SOLVENT CONTENTS: Less than 1%

SODIUM PENTABORATE PENTAHYDRATE {$NaB_5O_8 .5H_2O$} ASSAY

OBJECTIVE: To determine the purity of PSP
PRINCIPLE: The sodium borate composition can be expressed in terms of sodium oxide, boron trioxide and water equivalents. The PSP empiric formula ($NaB_5O_8$. $5H_2O$) can be expressed as $Na_2O$, $5B_2O_3$, $10H_2O$. $NaO_2$ content is verified by standard HCl titration. $B_2O_3$ content is verified by standard NaOH titration. The purity of PSP is calculated from the $B_2O_3$ content and depends on boron.

EQUIPMENT

400-mL beaker
9-cm watch crystal (3.5 inches)
Magnetic stirrer/hot plate
Stir bar
50-mL burette

25-mL burette

CHEMICALS

Hydrochloric acid 0.5 N (HCl)
Sodium hydroxide 0.5 N (NaOH)
Mannitol
Phenolphthalein as an indicator in a 1% alcohol solution
Methyl red as an indicator in a 1% alcohol solution
Methylene blue as indicator in a 0.25% aqueous solution

PROCEDURE

1. Dissolve 1.00+/-1 mg PSP into 100 mL purified water. Record the exact weight.
2. Add 5 drops of methyl red, add HCl 0.5 N from the 25-mL burette to acidify the solution until it becomes red. Add a 0.3 to 0.4 mL excess and record the final volume.
3. Boil for 3 minutes to eliminate $CO_2$, cool down and take volume to 150 mL.
4. Add one drop of methylene blue and titrate excess of HCl with NaOH 0.5 N. Record the volume.
5. Add to the neutral titrating solution of $Na_2O$, about 15 g mannitol, 5 drops phenolphthalein and titrate to end point with NaOH 0.5 N. Record the volume (see note).
6. Add a small quantity of mannitol (2-3 g) if the color of the solution fades continue with the titration.
7. Repeat step 6 until the change becomes permanent.
8. Estimate the acid and basic percentages.

$$\%\ Na_2O = \text{net volume (mL) of HCl 0.5 N X 1.550}$$

$$\%\ B_2O_3 = \text{net volume (mL) of NaOH 0.5 N X 1.741}$$

NOTE: When mannitol is added to the neutralized solution its color will change from green to purple. This color is due to the acidity of the complex mannitol-boric acid formed and it must not be confused with the purple color of the phenolphthalein change. Sodium hydroxide in the solution will turn the purple color into green and purple again when the solution is alkaline to phenolphthalein. The end point is reached when all boric acid has been transformed in sodium metaborate.

$$2H_3BO_3 + 2NaOH + 4H_2O = Na_2B_2O_4.\ 8H_2O$$

VALIDATION OF THE SODIUM PENTABORATE PENTAHYDRATE ($NaB_5O_8$ .5$H_2O$} ASSAY.

EQUIPMENT: The following material was used in validations and assays performed in the laboratory and are shown herein as examples.
Cole-Palmer stirrer/hot plate model 4658 (2 used for stirring the titration mixtures)
Corning stirrer/hot plate, model 6795-520 (to boil the test solutions).
25-mL Kimax$^R$ Schellbach Automatic Burette, Kimble 17170F to dispense HCl 0.5 N
50-mL Kimax$^R$ Schellbach Automatic Burette, Kimble 17170F protected with calcium oxide, to dispense NaOH 0.5 N
pH-meter model 220 with combination electrode semi-micro, No. 476540
Mettler analytical scale, model H54

CHEMICALS

Mannitol, EM Science, USP grade, No. MX0216

Orthoboric acid, EM Suprapur$^R$, No. 756-1, batch 9249

Disodium tetraborate anhydrous, EM Suprapur$^R$, No. 6309-1, batch 9118

Sodium hydroxide 0.5 N, prepared in solution by VWR company, batches 90060602 and 90072601 BTR

Hydrochloric acid 0.5 N, prepared in solution from RICAA, batch 90060601 BTR

Potassium biphtalate, Baker analytical reactive, standard acidimetry No. 2958-01, batch D18084.

Phenolphthalein, EM Science No. PX 0525-1, batch 9142

Methylene blue, Baker analytical reactive, No. Q475-03, batch D03728

Methyl red, sodium salt, Baker analytical reactive No. R086-02, batch D14708

Water was obtained from the Continental Water Systems Modulab unit, with a resistivity higher than 18 Megohm-cm.

PROCEDURE

For precision of the assay, 5 sequential reproduced determinations were run; the samples were dried at 50°C about 24-36 hours before titration.

For precision of the assay, the orthoboric acid and the disodium tetraborate Science Suprapur$^R$ grade EM, were stored in plastic bags containing a desiccator until used.

For the sensitivity and specificity studies, mixtures of dry powders were prepared immediately before the assays and were titrated without additional treatment to be used in the procedure for pentahydrated sodium pentaborate.

The weights of the samples were 1.000+/-0.0001 g unless otherwise indicated and were considered as 2.000 g for all calculations.

The standardization of sodium hydroxide was first tried with 1 g potassium biphtalate aliquots (KHP), but it was found that the best results were obtained with a larger quantity of KPH by determination (3 g by NaOH 0.5 N, 6 g by NaOH 1N), so that the final volumes were near to those used in unknown determinations. KPH was oven dried at approximately 115°C for 1 hour and stored in a desiccation until used.

CALCULATION:

Boron trioxide content

$$\% B_2O_3 = \frac{B_2O_3 \text{ weight in the sample}}{\text{Weight of the sample}} \times 100$$

$B_2O_3$ weight in the sample is obtained by multiplying ($B_2O_3$ equivalents in the sample) x ($B_2O_3$ equivalent weight)

$$B_2O_3 \text{ equivalent weight } \frac{PM\ B_2O_3}{2} \quad \frac{69.62}{2} = 34.81$$

$$B_2O_3 \text{ equivalents in the sample} = (\text{NaOH volume}) \times (\text{NaOH normality})$$

$$\% B_2O_3 = \frac{(\text{mL NaOH})(\text{NaOH normality})}{\text{weight of the sample in grams}} \times 3.481$$

Sodium oxide content

The calculations are the same as those for the $B_2O_3$, with the exception of:

$$Na_2O_3 \text{ equivalent weight} = \frac{61.98}{2} = 30.99$$

$$\% Na_2O = \frac{(\text{mL HCl})(\text{HCl normality})}{\text{Weight of the sample}} \times 3.4099$$

RESULTS:

Acid- base standardization

1. NaOH 0.5 N, batch 90072601 was titrated 5X against 3 grams KPH.
2. HCl 0.5 N , batch 90060601, was titrated 3x against NaOH 0.5 N, batch 90072601; the following results were obtained
0.501 N; 0.500 N; 0.502; average = 0.501 N
3. NaOH 0.5 N, batch 900609602, was titrated 6X against HCl 0.5 N, batch 90060601, the following results were obtained
0.501 N; 0.501 N; 0.501 N; 0.501 N; 0.501 N; 0.501 N; average = 0.501 N

PSP purity

$$\text{PSP purity} = \frac{\%B_2O_3 \text{ in the sample} \times 100}{\%B_2O_3 \text{ in PSP}}$$

$$\%B_2O_3 \text{ in PSP} = \frac{B_2O_3 \text{ equivalent weight} \times 100}{\text{PSP equivalent weight}}$$

$$= \frac{5(69.62)}{2(295.2)} = 59.0\%$$

$$\text{PSP purity} = \%\ B_2O_3 \text{ in the sample } X\ 1.695$$

USES OF PSP

The first approach was to find an antibiotic similar to penicillin, but with no undesired effects such as hypersensitivity, hence during the first phase different fungi strains were used for making the drug (very large and complex process that currently and after many assays and studies, was reduced to just few days); after the assay with animals, the dose and safety interval for humans were determined; toxicity testing in different animal species (mice, rats, dogs, monkeys, etc.), allowed its use in healthy human beings and later on, in sick human beings showing excellent results, mainly in bacterial diseases in the absence of harmful side effects at any level, such as blood, renal, hepatic or any other body organ. What drastically changed the use of this drug was its use in a 2-year old child with a diagnosis of meningoencephalitis of viral etiology, mentioned in this descriptive report, which allowed us to notice the use of the drug in other different diseases of viral etiology with excellent results.

An evaluation of the antibacterial activity conducted by Microbiology Specialists of Houston Texas was performed in vitro. In this study, an aqueous solution of the PSP experimental drug was used to saturate paper discs that were lyophilized and later applied over an agar surface previously seeded with the test microorganisms, five PSP dilutions were tested 40 μg/mL, 400 μg/ml, 4 mg/mL, 40 mg/mL and 400 mg/mL, against a 400 mg/mL NaCl control. Fourteen bacterial species were tested and all of them were sensitive to the effects of 400 mg/mL PSP in vitro (Table 3).

TABLE 3

| BACTERIAL SENSITIVITY TO PSP | | | | | | |
|---|---|---|---|---|---|---|
| Bacterial species | Disc number | | | | | |
| | 1 | 2 | 3 | 4 | 5 | 6 |
| Streptococcus *pneumoniae* | 6 | 6 | 23 | 6 | 6 | 6 |
| *Streptococcus pyogenes* | 6 | 6 | 20 | 6 | 6 | 6 |
| *Streptococcus aureus* | 6 | 6 | 25 | 6 | 6 | 6 |
| *Neisseria gonorrhoeae* | 6 | 6 | 34 | 6 | 6 | 14 |
| *Neisseria meningitides* | 6 | 6 | 26 | 6 | 6 | 6 |
| *Pseudomona aeruginosa* | 6 | 6 | 23 | 6 | 6 | 6 |
| *Shigella dysenteriae* | 6 | 6 | 20 | 6 | 6 | 6 |
| *Shigella flexneri* | 6 | 6 | 21 | 6 | 6 | 6 |
| *Salmonella tifi* | 6 | 6 | 21 | 6 | 6 | 6 |
| *Escherichia coli* | 6 | 6 | 20 | 6 | 6 | 6 |
| *Proteus vulgaris* | 6 | 6 | 26 | 6 | 6 | 6 |
| *Haemophilus influenzae* | 6 | 6 | 23 | 6 | 6 | 6 |
| *Brucella suis* | 6 | 6 | 27 | 6 | 6 | 6 |
| *Brucella suis* | 6 | 6 | 30 | 6 | 6 | 6 |
| *Klebsiela* | 6 | 6 | 25 | 6 | 6 | 6 |
| *Micobacterium leprae* | gd | gd | nd | gd | gd | gd |
| *Micobacterium tuberculosis* | gd | gd | nd | gd | gd | gd |
| gd = good development; nd = did not develop | | | | | | |

The figures that link the strains with the disc number correspond to the inhibitory halos in mm.

The numbers under the Disc heading represent the following dilutions:

PSP: 1= 4 mg/mL; 2= 40 mg/mL; 3= 400 mg/mL; 4= 40 μg/mL; 5= 400 μg/mL

NaCl 6= 400 mg/mL (control)

The results obtained suggest that PSP can be used as an antibiotic.

After in vitro testing, we performed an in vivo testing in mice, rats, monkeys, and dogs, the result of this were used to set dosage for use in humans.

IN VIVO TOXICOLOGY TESTING IN ANIMALS ACUTE AND SUB-ACUTE TOXICITY ASSAYS IN MICE.

To determine toxicity of a single dose and to find out the maximum tolerated dose (MTD), 1000 mg BPCS/kg daily doses were administered intravenously to mice. All animals were normal until day 14, no important injuries were observed during necropsy. The average mice did not showed a loss of weight, but one animal showed a loss of weight of 3.6 g between days 7 and 14. The loss of weight of this animal was considered inexact since its body weight increased between days 0 and 7, and due to the lack of clinical signs of important tissue alterations in the necropsy. These findings showed that PSP in the test conditions has low toxicity in mice. Since mice remained healthy with a 1000 mg BPCS/kg dose, this dose was considered as the maximum tolerable dose (MTD) for mice. MDT was administered orally via a catheter or by intraperitoneal infusion for 20 consecutive days. PSP was administered into drinking water at a dose of 1.2 or 3 PSP mg/mL for 20 consecutive days. The toxicity sign in this kind of assay is a decrease in body weight in animals getting PSP for a 20-day time period of the experiment. The mean weight was calculated for each group; only the group treated with PSP in drinking water at a 3-mg/mL dose showed a higher increase in body weight than ribarvin controls.

At a 3 mg PSP/mL water dose, the treatment group showed an increase in body weight of 41.1% in comparison with the ribarvin control groups. All the treatment groups showed an increase in weight greater than 71%.

ACUTE TOXICITY ASSAYS IN RATS

Single-dose toxicological assays were performed in rats. The dose interval was between 100 mg PSP/kg and 4000 mg PSP/kg administered orally by catheter. At a 4000 mg/kg dose, male rats showed a loss of weight of 11%. There were not observed signs of weight loss in the female group. The dose of 4000 mg/kg was chosen for the consecutive daily dosage. Alter two consecutive 4000 mg/kg doses, none of the test animals survived. The dose was reduced to 1000 mg/kg and was administered to another group of rats (3 of each gender) for three days. No abnormal signs were observed in these three days; the dose was increased to 2000 mg/kg. All rats died after four days of consecutive treatment at 2000 mg/kg. These results suggest that MTD for PSP in rats is around 1000 mg/kg, this is consistent with the MDT dose for mice.

ACUTE AND SUB-ACUTE TOXICITY ASSAYS IN MONKEYS.

Acute and sub-acute toxicity assays were performed in monkeys. Control and test animals were administered with a dose of 500 mg PSP/kg and 250 mg PSP/kg. At both dosages, animals showed a toxicity response characterized by vomiting and/or anorexia after each dose. MTD for monkeys was determined at 100 mg/kg; dosing continued for 14 consecutive days. Once the dose regimen ended, a necropsy was performed on all the animals. No important injuries were identified.

CONCLUSION

The results of the acute and sub-acute toxicology assays suggest the following MTD:

In mice 1000 mg PSP/Kg bodyweight
In rats, close to 1000 mg PSP/kg bodyweight
In monkeys 100 mg PSP/kg bodyweight
Based in these results, it is considered that is reasonably safety to use a dose between 10-20 mg/kg in humans.

Boric acid is not considered as an acute poison.

After intake or absorption into the blood of a large quantity of boric acid, symptoms may appear between 24 to 72 hours. Borates are cleared in urine, 70% of it during the first 24 hours.

After two years of conducting assays consisting in feeding rats and dogs with PSP in the food, no signs of boron accumulation in the body were observed. There are no side effects with PSP at 0.31% in the diet.

PHARMACOLOGICAL STUDIES

Rauscher Murine Virus (Rauscher MuLV) System *in vivo.*

Splenomegaly was observed on infected animals with the Rauscher MuLV virus, seven days after the infection. The reason and magnitude of the spleen weight augmentation over time depends on the dose of the administered virus, and can be modified by agents that directly or indirectly interfere in the viral replication.

Mice were inoculated with the Rauscher MuLV and treated with PSP administered by drinking water at a 1.2 or 3-mg/mL dose, via a catheter at doses of 250, 500 or 1000 mg/kg/day or intravenously at doses of 250, 500 or 1000 mg/kg/day. Typically, the infection resulted in splenomegaly in animals that received no treatment. Results showed that PSP at 3 mg/mL doses in drinking water reduced 72% the spleen size; rivabirin reduced 74% the spleen size, in comparison with the infected animals that received no treatment.

Pharmacological properties In vitro:

Toxicity studies were performed to evaluate the PSP effect over "fresh" T cells and over H9 cells. PSP dilutions were added into two cellular cultures; the results showed that PSP at 333 μg/mL and 1000 μg/mL was toxic for the cells. The cellular development was affected and apparent cytopathic effects increased.

The in vitro methods used to determine the PSP pharmacological activity show antibacterial activity, but do not show antiviral or virucide activity. It has been demonstrated that PSP protects mice infected with Rauscher MuLV by reducing splenomegaly size. In the light of the absence of antiviral activity or virucide activity in vitro, a possible mechanism of action of PSP is immunomodulation.

IN VIVO ASSAYS WITH HUMANS

The above studies allowed us to identify a dose of 10 to 20 mg/kg bodyweight and we tested this dosing interval with a wide range of bacterial and viral diseases, for example: Laringo-trachea bronchitis, herpes Zoster, hepatitis B, chicken pox, sepsis, among others.

<u>Laringo trachea bronchitis</u>:

A seven-month male child of 7 kg weight, eutrophic, with laringo trachea bronchitis symptoms. Poor general conditions, restless, crying, cyanotic (+), with Silverman Andersen 7 (nasal flapping, intercostals pull, moans, xiphoideal retraction, toracoabdominal asynchrony), polynesic and hiporexic.

50 mg PSP were administered orally each 12 hours, equivalent to 15 mg/kg/day. Recovery started within the first hours and there was no need of hospitalization; after 48 hours the symptoms disappeared. The child received treatment for one more week.

<u>Herpes Zoster</u>

Male patient, 54 years old, had suffered from pain at the right side of thorax for one week, the costal zone showed very painful blisters following the nerve path, had previously been treated with steroids and analgesics, showed no improvement. Treatment started with PSP orally at 500 mg each 12 hours, along with analgesics and B complex (IM) each 72 hours. 24 hours after the treatment started, a remarkable improvement was shown, after one week of initiated treatment, the patient was asymptomatic. The patient was treated with 500 mg per day for 3 more weeks.

<u>Hepatitis "B"</u>

A 7-year old girl, started symptoms as a mild alteration of her general condition, mild jaundice, asthenia, adynamia, hyporexia, low fever, acoholia, choluria, mild pain at right hypochondrium, inconspicuous hepatomegaly, lab tests showed alterations of the hepatic function tests, with an increase of bilirubin level, an increase of ALT, AST, and alkaline phosphatase.

Oral administration of 100 mg PSP every 24 hours for one month. In the first few days after treatment initiated a gradual decrease of all the symptoms was observed, the symptoms disappeared within 10 days and one month later the laboratory exams showed normal levels.

Chicken pox

A 3-year old girl showed the characteristic exanthema with papules, vesicles and pustules, practically covering her entire body, mild alteration of her general condition, mild asthenia, and adynamia, low fever and hyporexia.
Treatment started three days after the onset, with 100 mg PSP/24 hours, the pathological symptoms disappeared. 72 hours later, the patient was practically asymptomatic with the exception of some dermal lesions, which disappeared two days later.

Sepsis

26 years old female patient, with sepsis. Record of cesarean operation 48 hours before her hospitalization, very bad general condition, 40°C fever, hypotension, tachycardia, purulent bloody exudates from the surgical wound and transvaginal. Treatment was initiated with 150 mg PSP (IV) every 8 hours and 1.5 g via an intravenous solution every 24 hours with continuous dripping, together with penicillin, and gentamicin via systemic route. 24 hours later the infection signs started to disappear, she became asymptomatic 72 hours later, and she was discharged from hospital at day 5 without complications.

Meningoencephalitis

Two-year old boy, admitted in hospital in very bad general conditions, no fever, neurological symptomatology: Data of meningeal irritation, neck stiffening, Kernig (+), Brudzinsky (+), Babinsky (+), generalized hyper reflection, augmented muscular tonic-clonic convulsions, stupor and later in coma; diagnosis: Viral meningitis, diagnosed by spinal fluid analysis. Treatment initiated with 150 mg PSP IV (20 mg/kg/day) every 12 hours, the patient started recovery from unconsciousness and general symptoms started to disappear, on day 5 after treatment initiated, the patient was discharged from hospital, the only sequel of the neurological process was paresis of VI cranial pair, which reverted three months after the patient was discharged from the hospital. Treatment at home was 100 mg PSP IM every 12 hours for one week and afterwards the same dose every 24 hours for two more weeks. Patient did not report any other subsequent problem and has a normal life.

Hepatitis C

32 years old male patient, with diagnosis of hepatitis "C". Symptomatology: hepatomegaly, moderate alteration to his general condition, astenia, adynamia, cephalea, myalgia, no jaundice, low fever, and hyporexia. Treatment initiated with 500 mg PSP every 24 hours (15 mg/kg/day), for 15 months; the whole process reverted including normal laboratory tests (ALT, AST, and alkaline phosphatase, etc.) At present the patient is asymptomatic, has gained weight and does not shows any symptom.
The above-mentioned results encouraged us to test the drug within the area of viral diseases, so we tested the drug with AIDS patients obtaining the following results:

> We decided to set up clinical assays to evaluate the PSP therapeutic utility against AIDS.
> PSP is water-soluble, so it can be directly used to obtain the drug at the required concentration without having to obtain the acid salt form to be administered.
> The PSP is useful to formulate pharmaceutical compositions with pharmaceutically accepted vehicles in several pharmaceutical presentations, such as tablets, capsules, lozenges, and solutions for drops, syrups, ovules, suppositories, gels, lotions, and ampoule vials, for its oral, nasal, ophthalmic, otic, local, intracavitary, vagina, rectal, dermal, capillary, intramuscular, intravenous, intradermic, subcutaneous administration or any other means well suited for its administration to and absorption by the body.

CLINICAL ASSAYS

The objectives of the clinical assays were the following:

1. To identify the required drug dose to modify the disease process.
2. To establish the risks of such dose.
3. To keep controls of the objective and subjective conditions; and
4. To establish the PSP benefits and efficacy.

These studies included antibody studies, virus antigen control studies and complete cell blood counts, including

lymphocyte phenotype.

The first study included 18 patients. All subjects were positive for HIV-1 antibodies and Walter Reed (WR) classification WR2 to WR6 at the beginning of the study, as per Table 4. Nine of the 18 subjects were rated WR2 and WR3 (without detectable immunological damage), two patients were rated WR5 and the remaining five patients were rated WR6.

For a five-month period, patients were administered with the drug by oral administration, two to three times a day, the total dose being 500 to 2500 mg per day, depending on CD4 counts at the beginning of the treatment. After this period, treatment was discontinued and the subjects had follow-up visits, and regular laboratory and clinical tests were performed. These studies are ongoing.

A remarkable favorable response pattern to PSP treatment in all HIV infected individuals was observed after beginning of the treatment. All patients developed one or more of the following side effects: Loss of weight, sweating, depression, minor skin scaling on hand palm and feet, diarrhea, or constipation and headache. During the same period, a reduction of the total lymphocytes was observed, specifically CD8 and CD4 in many of the patients. From week 8 to week 12 of the treatment, an interruptions of the symptoms was observed and a significant increase of total lymphocytes was reported, especially CD8 (greater than normal limit for non-infected humans). A similar pattern of activated T cell markers, HLA-DR, was observed. A second marker for T cells activation (IL-2) could not be established in this study due to the age of the cells before the analysis could be performed.

Five months after termination of treatment, 14 out of 18 patients showed good health. Three patients died within the first 21 weeks of treatment, two of them due to an advanced Kaposi sarcoma (KS) and the third one due to an advanced malnutrition. The fourth patient who had neurological manifestations (secondary AIDS dementia before treatment) responded favorably during 2 to 3 months, but later on his neurological condition returned. He was maintained with PSP (500 mg/day) for five months after treatment, and finally died one year after he started the treatment.

TABLE 4 WALTER REED (WR) STAGING CLASSIFICATION

| Condition | HIV or viral antibody | chronic lymphadenopathy | T helper/ mm | Delayed hypersensitivity | Thrush (*) | Opportunistic infections |
|---|---|---|---|---|---|---|
| WR0 | - | - | >400 | Normal | - | - |
| WR1 | + | - | >400 | Normal | - | - |
| WR2 | + | + | >400 | Normal | - | - |
| WR3 | + | +/- | <400 | Normal | - | - |
| WR4 | + | +/- | <400 | P | - | - |
| WR5 | + | +/- | <400 | C and/or thrush | C and/or thrush | - |
| WR6 | + | +/- | <400 | P/C | + | + |

(*) Oral monillasis or oral candidiasis
P = Loss, without response.
C = Along with oral monillasis.

The four patients that did not respond favorably had a CD4 count of less than 40 (absolute values) when the treatment started, the normal CD4 count is more than or equal to 400. In spite of the last failure in these four patients to respond to treatment, they experienced an improvement of quality of life at least for four months, along with an improvement of appetite and decrease of symptoms for the initial months. The KS lesions responded favorably and remained dry during the weeks 2 to 8 of treatment. Nevertheless this response was temporary and lesions became more aggressive before the death of the two patients with KS.

Regarding the 12 patients that showed good health, five months after the PSP treatment was discontinued, two characteristics in the response patterns were observed. Seven of them tested negative to the presence of the virus and CD4 cells were significantly increased (statistically these increases occurred randomly in 1% of the cases). These patients maintained or increased their bodyweights in the nine-month period after the beginning of the treatment. The five remaining patients apparently had good health, even though they remained positive to the virus and their CD4 cells decreased or remained constant. Furthermore, the anti-HIV antibody activity determined by ELISA commercial tests raised from 2 to 12 times in 10 of the 16 surviving patients, for this 9 months.

All patients positive to the virus will continue under control to determine if the test becomes negative (the virus is no longer detected) and to identify the time when the CD4 cells increase.

CONCLUSION OF THE CLINICAL ASSAY:

PSP seems to modify the process of the disease under a 2500 mg dose per day. Nevertheless, strong side effects were observed in some patients. Four patients that began with less than 40 CD4 cells did not respond favorably to the therapy. One of them was asymptomatic during the treatment and seemingly with good health during five months after the conclusion of the treatment. 14 of the 18 patients stayed alive 40 weeks after the treatment was discontinued. The 7 patients that showed a significant increase in CD4 cells during the treatment, remained negative to the virus and to the p24 antigen during the period subsequent to the treatment, and their CD4 cells after the treatment remained at normal titers. The four remaining patients who responded favorably, showed a decrease in CD4 cells after the treatment, recently they initiated a second treatment with PSP. These patients will be carefully observed. It should be mentioned that these four patients have remained p24 positive. A patient previously treated with AZT before the PSP treatment, did not show any change during the 40-week follow-up after the treatment, while another patient developed a pulmonary infection with Pneumocystis carinii (PCP), and responded partially with sulphametoxasol; later on he died due to that same infection. Both patients were treated a second time with PSP. A significant result to this first assay is that asymptomatic patients kept asymptomatic for a five-month period after the treatment was suspended, in contrast to the immediate deterioration of patients whose AZT treatment was withdrawn. First clinical assays require of further investigation to establish a therapeutically effective lower dose of the drug to modify the disease process.

The observations of these assays suggests that a daily 2500 mg dose causes side effects in some patients, as severe rash, depression, diarrhea, and gastric issues with an average of 3.1 kg loss of weight. In most of the patients it was also observed a decrease in total lymphocyte titer, mainly CD4 and CD8 during the first 6 to 8 weeks of treatment. For example, in one patient CD4 decreased from 269 to 66 in the first 8 weeks of treatment. The in vivo and in vitro obtained results suggest that the mechanism of action of PSP is by immunomodulation. In clinical assays we observed a decrease in CD4 and CD8 typically followed by CD8 stimulation and later by CD4 stimulation. Actually, this mechanism of action is under study. These clinical observations encourage us to perform other clinical assays. A medical record form was designed and the Epi-info Version 3 was implemented. Epi-info is a statistical software designed by the Centers for Disease Control at Atlanta, Georgia, U.S.A for these kind of clinical assays.

The study protocol for the second group was modified. The modifications included a decrease in the drug dose to a maximum of 1500 mg per day vs. 2500 mg per day, a series of cutaneous reaction tests before and after the treatment and additional clinical laboratory tests Clinical assays included 19 HIV-1 positive patients, out of which 17 were classified from WR4 to WR6 and the remaining two were classified WR3. All patients WR4 to WR6 were immunocompromised, which means they have partial or null response to the antigen series of the cutaneous reaction tests, which is a direct measure of the cell-mediated immune response.

Each patient was administered with PSP orally for a four-month period with a 500 to 1500 mg per day dose. The side effects were observed after the seventh week of treatment. In these studies, the side effects were similar, but less severe than those observed in the first study. For example: Due to smaller doses, a less severe weight loss was observed in this group (the average of weight loss was 0.5 kg in comparison with 3.1 kg of the first assay). The clinical observations in the second group were recorded every two weeks during the study. Records on these observations up to the date before the treatment, and 12 weeks before the beginning of the treatment, showed that no patient was free of symptoms at the beginning of the assay; 14 had 3 or more symptoms. After 12 weeks of treatment, 13 patients showed improvement without subjective complains, no patient showed more than three symptoms. It must be observed that problems such as night sweating, loss of weight, nausea, vomiting, epigastric pain, HSV (herpes simplex virus) disappeared during the first few weeks of treatment.

Ii is of particular concern a report about a patient with early KS. KS was detected in this patient after screening and first blood sample. Just before initiating the therapy with PSP. This patient was initially administered with a combined treatment of PSP and vincristine sulphate. After the twelfth week of treatment, KS lesions did not increase or were not confirmed by pathology and remained negative for five months. The laboratory findings and/or the medical observations confirmed the clinical improvement. Signs such as adenomegaly and monillasis were solved within 12 weeks. The frequency of lymphadenopathies decreased as of the third observation at the beginning of the study to nearly the end of the study, likewise monilliasis frequency decreased from 12 to 1.

In contrast to the group of the first clinical assay, there were no significant changes from the initial CD4 and CD8 counts during and up to the conclusion of the second assay. On the other hand, there was a statistically significant increase in lymphocyte T activity detected by HLA-DR and IL-2 markers. Both cell types decreased 3 weeks after the beginning of the treatment. From that date on, the cell titers of both phenotypes (HLA-DR and IL-2) have increased and reached a plateau apparently beyond the normal values.

A series of cutaneous reaction tests were performed to all patients involved in the second study before initiating treatment and five months after it initiated. Nine out of 19 patients developed a positive response to one or more of the antigens of the cutaneous tests during the five-month treatment, suggesting the recovery of the cell-mediated immune response to one or more previously confronted infectious diseases.

Three patients in the second clinical study died. They started respectively with CD4 titers of 30, 10 and 10. The first patient developed PCP and responded briefly to the treatment with trimetoprim and sulphametoxazole for pulmonary infection, but quickly died due to unknown causes. The second patient developed dementia and malnutrition with emancipation and consumption. The third one developed brain toxoplasmosis. The first two patients died at week 12 of treatment and the third one died at week 25 after the end of the treatment.

Two HIV-antibody negative adult subjects were included in the second clinical assay as normal controls. Each patient was administered with two PSP capsules twice a day (1000 mg a day) for 6 to 8 weeks respectively. During week 6 of treatment, the first normal control had a minor gastric discomfort at week 2 and lost about 3 kg of weight that the patient attributed to personal family issues. The second control received PSP for eight weeks, with no side effects. Based on these limited observations, it seems that PSP does not produce toxicity or has minimal toxicity in normal adults.

CONCLUSION OF THE SECOND CLINICAL ASSAY

Based on the preliminary evaluation of the response of the patients in the second clinical assay, it is concluded that PSP seems to be effective and safe for HIV-1 positive asymptomatic individuals and whoever has a clinical progress of ARC (AIDS related complex) or AIDS (WR4, WR5 y WR6). It seems that signs and symptoms disappeared after week 12 of treatment. Nine out of 16 surviving patients continued a treatment with PSP for 4 months; the seven remaining patients were administered with one 500 mg capsule per day for two months. The subjective and objective controls of the patients receiving from 500 mg to 1500 mg PSP daily, suggest that the side effects are minimal.

The actual concept of the virological and immune response of AIDS patients treated with high PSP dosages (2500 mg/day) is as follows:

1.- At the beginning of the PSP treatment, the titer of CD8 and T cells carrying HLA-DR markers increased more than normal titers and declined to normal titer after the end of the treatment.

2.- Patients that do not manifest the presence of the virus (are negative to the virus) experience an increase of anti-HIV antibody activity according to CD4 cell titer increases. The ability of the CD* cells to inhibit the replication of the virus has been previously reported.

Upon decreasing the dose in the second clinical assay (maximum 1500 mg/day), main changes in lymphocyte phenotypes are observed in activated T cell populations, HLA-DR or IL-2. Most of the reports show that the number of HLA-DR cells decreased as the disease evolved. This suggests that the lymphocyte T activation as response of the PSP therapy in WR4, WR5 and WR6 patients can serve as a marker for specific immunomodulation. It is expected to perform subsequent assays about the performance of these cell populations.

It is of interest to mention that two patients of the second clinical assay, who were classified as WR3, had an increase of DC4 and CD8 cells after week 18 of treatment. In these two patients the changes in T cell activation were variable. It seems that the lymphocyte population responding to PSP therapy may depend more on the stage of the disease than on the dose of the drug.

It is important to note that in one patient KS lesions seemed to resolve as response to the combined PSP and vincristine therapy, thus it is worthy to study other combination therapies.

The advantages of PSP in the therapy of infectious diseases include the absence of significant toxic side effects, efficacy against infections that had not responded to other drugs, and the ability to treat patients with chronic viral infections such as HIV and viral infectious processes, such as, measles, chicken pox, herpes Zoster, hepatitis B, viral meningoencephalitis, hepatitis C, influenza, laryngotracheitis, pharyngitis, bronchitis, broncheolitis, pneumonia, common cold, gastrointestinal diseases from viral etiology, herpes simplex and Zoster, mumps, smallpox, lymphogranuloma, cytomegalovirus, dengue, general encephalitis, rabies, poliomyelitis, and Ebola.

The difference between TD4 and TD8 concentrations at the initial stages and the final stages suggests that PSP has an immunomodulator effect, namely it modulates the immune system, helps the organism to self-recover, find the equilibrium, and reestablish a healthy condition.

We believe that due to the low toxicity level of PSP, due to the unique mechanism of action, and due to its potential use in combination with other cellular antiviral activity, PSP can take a unique and highly competitive position in the field of AIDS therapy and treatment of other bacterial and viral diseases.

## Claims

**1.** An improved synthesis to obtain pentahydrated sodium pentaborate, **characterized in that** the synthesis consists of the following steps:

a) gently dissolving the reactants in purified water within an adequate container by gradually adding them alternately, while stirring at a temperature between 59 and 61°C;
b) stirring the solution;
c) allowing the reaction to occur until the solution gets clear;
d) filtrating the solution;
e) growing crystals;
f) separating the formed crystals from the filtrate;
g) refrigerating the finished product;
h) drying the crystals; and
i) packaging the product.

**2.** The improved synthesis to obtain pentahydrated sodium pentaborate of the claim 1, **characterized by** the reactants being orthoboric acid and pentahydrated sodium pentaborate, and their weights being calculated at 100% purity.

**3.** The improved synthesis to obtain pentahydrated sodium pentaborate of claim 2, **characterized in that** for one kilogram of the finished product, 680.0 g of orthoboric acid and about 700.0 g of sodium tetraborate decahydrate both 100% pure are needed; these reactants being dissolved into about 1600 mL purified water at about 59 to 61°C.

**4.** The improved synthesis to obtain pentahydrated sodium pentaborate of the claim 3, **characterized in that** the reactants are added to water little by little and alternately, while stirring at 200 RPM.

**5.** The improved synthesis to obtain pentahydrated sodium pentaborate of the claim 1, **characterized in that** the temperature must not exceed 61°C.

**6.** The improved synthesis to obtain pentahydrated sodium pentaborate of the claim 1, **characterized in that** once the reactants are completely dissolved, the solution is removed from heat and filtered to eliminate any remaining solids.

**7.** The improved synthesis to obtain pentahydrated sodium pentaborate of the claim 1, **characterized in that** the temperature of the solution is taken to 21°C while stirring at 200 RPM, to make crystals grow, the crystal may be grown by any means, but the cooling method is preferred.

**8.** The improved synthesis to obtain pentahydrated sodium pentaborate of the claim 7, **characterized in that** the temperature of the solution is lowered down to 10-12°C while slowly stirring at 150 RPM.

**9.** The improved synthesis to obtain pentahydrated sodium pentaborate of the claim 7, **characterized in that** the container with the solution and crystals is maintained at 6°C for 18-20 hours.

**10.** The improved synthesis to obtain pentahydrated sodium pentaborate of the claim 10, **characterized in that** the crystals are removed and dried.

**11.** The improved synthesis to obtain pentahydrated sodium pentaborate of the claim 1, **characterized in that** the step of packaging is performed in plastic containers with screw cap.

**12.** The product obtained by means of the improved synthesis of claims 1 to 12.

**13.** The product of claim 13, which is pentahydrated sodium pentaborate ($NaB_5O_8 .5H_2O$).

**14.** The pharmaceutical compositions **characterized in that** pentahydrated sodium pentaborate is the only active component with at least one pharmaceutically accepted carrier or diluent.

**15.** The pharmaceutical compositions of claim 14, **characterized in that** its pharmaceutical presentation maybe tablets, capsules, lozenges, and solutions for drops, syrups, ovules, suppositories, gels, lotions, and ampoule vials, for its oral, nasal, ophthalmic, otic, local, intracavitary, vagina, rectal, dermal, capillary, intramuscular, intravenous, intradermic, subcutaneous administration or any other well suited means for its administration to and absorption by the body.

**16.** The pharmaceutical compositions of claim 15, **characterized in that** the preferred pharmaceutical presentation is

capsules.

**17.** Pharmaceutical compositions **characterized by** having as active compounds pentahydrated sodium pentaborate and at least one or more active compound, along with at least one pharmaceutically accepted carrier or diluent.

**18.** The use of pentahydrated sodium pentaborate, obtained by means of the method mentioned in claims 1 to 11, to manufacture a drug product for the treatment of diseases caused by bacteria.

**19.** The use of pentahydrated sodium pentaborate, obtained by means of the method mentioned at claims 1 to 11, to manufacture a drug product for the treatment of diseases caused by virus.

**20.** The use of pentahydrated sodium pentaborate obtained by the method of the claims 1 to 11, according to claim 18, wherein the bacteria causing the diseases are, for example, *Streptococcus pneumoniae, Streptococcus pyogenes, Streptococcus aureus, Neisseria gonorrhoeae, Neisseria meningitides, Pseudomona aeruginosa, Shigella dysenteriae, Shigella flexneri, Salmonella typhi*, *Escherichia coli, Proteus vulgaris, Haemophilus influenzae, Brucella suis, Staphylococcus, Klebsiela, Mycobacterium leprae, Mycobacterium tuberculosis.*

**21.** The use of pentahydrated sodium pentaborate obtained by the method of claims 1 to 11, according to claim 19, wherein the diseases caused by virus are for example chronic viral infections, such as HIV, and acute viral infectious processes such as measles, chicken pox, herpes Zoster, hepatitis B, viral meningoencephalitis, hepatitis C, influenza, laryngotracheitis, pharyngitis, bronchitis, broncheolitis, pneumonia, common cold, gastrointestinal diseases from viral etiology, herpes simplex and Zoster, mumps, smallpox, lymphogranuloma, cytomegalovirus, dengue, general encephalitis, rabies, poliomyelitis, and Ebola.

**INTERNATIONAL SEARCH REPORT**

International application No.
PCT/ MX 2005/000079

A. CLASSIFICATION OF SUBJECT MATTER

*A61K33/22 (2006.01)*

According to International Patent Classification (IPC) or to both national classification and IPC

B. FIELDS SEARCHED

Minimum documentation searched (classification system followed by classification symbols)

A61K

Documentation searched other than minimum documentation to the extent that such documents are included in the fields searched

Electronic data base consulted during the international search (name of data base and, where practicable, search terms used)

CIBEPAT, EPODOC, WPI, CAS

C. DOCUMENTS CONSIDERED TO BE RELEVANT

| Category* | Citation of document, with indication, where appropriate, of the relevant passages | Relevant to claim No. |
|---|---|---|
| X | WO 9106304 A (AMERICAN BIOTECH RESOURCES, INC) 16.05.1991; the whole document. | 12-21 |

☐ Further documents are listed in the continuation of Box C.  ☒ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13th December 2005 (13.12.05) | 15th December 2005 (15.12.05) |
| Name and mailing address of the ISA/ S.P.T.O. Facsimile No. | Authorized officer Telephone No. |

Form PCT/ISA/210 (second sheet) (July 1992)

**INTERNATIONAL SEARCH REPORT**
Information on patent family members

International Application No
PCT/ MX 2005/000079

| Patent document cited in search report | Publication date | Patent familiy member(s) | Publication date |
|---|---|---|---|
| WO 9106304 A | 16.05.1991 | CA 2072619 A<br>AU 6961191 A<br>EP 0502978 A | 01.05.1991<br>31.05.1991<br>16.09.1992 |

Form PCT/ISA/210 (patent family annex) (July 1992)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 9006331 W
- WO 9106304 A, Dressman
- US 900633 W